# EUROPEAN PATENT APPLICATION

(11) **EP 4 414 989 A1**
(43) Date of publication of application: **14.08.2024**
(21) Application number: 24275008.1
(22) Date of filing: 07.02.2024
(51) Int. Cl.: G16B 25/10, G06N 20/00, G16B 40/20

(54) **METHODS**

(30) Priority: 07.02.2023 GB 202301739
(71) Applicant: Curenetics Ltd, Essex RM12 6BA (GB)
(72) Inventor: Adeleke, Sola, Hornchurch, RM12 6BA (GB); Ben-Ali, Ferid, Hatfield, AL10 9AB (GB); Mporas, Iosif, Hatfield, AL10 9AB (GB); Braoudaki, Maria, Hatfield, AL10 9AB (GB)
(74) Representative: Potter Clarkson

(57) **Abstract**

The invention relates to methods for generating a lower-dimensionality vector for training a classification model and uses thereof.

## Description

### Field of the Invention

The invention relates to methods for generating a lower-dimensionality vector for training a classification model and uses thereof.

### Backaround to the Invention

Medical treatments for cancer are becoming increasingly expensive. For example, some cancer therapies can cost in excess of £100,000 per patient, per year. Such treatments may not be equally effective on all patients with the same cancer types. Furthermore, it is sometimes difficult to identify and correctly diagnose a patient's cancer due to diagnostic limitations of current tools. By the time a patient has clearly identifiable symptoms, the illness may have progressed to a stage where it is difficult, expensive or in the worst cases impossible to treat.

In many cases different cancer types may have similar clinical manifestations, making them difficult to correctly identify or distinguish from each other. One such example is lung cancer, which may manifest as small cell lung cancer (SCLC) or non-small cell lung cancer (NSCLC) types, including large cell lung cancer (LCLC), lung adenocarcinoma (AD) and squamous cell carcinoma (SCC) of the lung. The most effective treatment pathway broadly differs for these lung cancer types.

It is known that there is a correlation between an individual's genes and their risk of developing certain types of conditions. Early signals or patterns of differentially expressed genes could help indicate whether a tissue is diseased or healthy even before clinical manifestation. Therefore, in recent years there has been an interest in using genomic data (information about the gene expressions encoded in a person's DNA) to classify and identify cancer. There have been attempts to apply statistical regression methods to analyse genomic data and train classification models. The classification models are calculated based on existing genomic training data, and then used to classify new genomic data.

Classification models can be used to categorise patients into cohorts based on patient data. The model may be used to categorise patients into cohorts according to their risk of developing a particular type of cancer. Alternatively, the model may be used to categorise patients into cohorts based on which type of cancer the patient is most likely to have.

In either case, by classifying patients more optimally into cohorts, patients can be better matched to treatments. This improves patient outcomes, by ensuring that the optimal treatment is assigned against a patient's particular type of cancer. In addition, healthcare cost efficiency is improved, by matching expensive therapies to the cohorts where they will be most effective. More optimal categorisation is also advantageous when developing new cancer treatments. When developing a new treatment for a specific kind of lung cancer, it may be difficult to evaluate the effectiveness of the treatment if that lung cancer cannot easily be distinguished from other types of cancer. It will be more expensive and time-consuming to identify patients with the appropriate kind of cancer in order to conduct clinical trials, and incorrectly diagnosed patients on the trial with other types of cancer may skew the results.

However, such classification models are limited by the difficulty of using genomic data. As is known in the art, statistical regression methods are most effective when the number of training samples in the training dataset is large relative to the number of features in the dataset. The more features, the more training samples required to perform accurate statistical regression analysis. This is sometimes referred to as the "curse of dimensionality". In the case of genomic data, the number of features is very large compared to the size of the dataset. The DNA of each individual contains a unique combination of tens of thousands, if not millions, of gene expressions and other genomic identifiers. Even if the dataset itself contains genomic data training samples from thousands of individuals, the number of training samples will still be low compared with the number of features.

Acquiring sufficiently large and appropriately populated genomic datasets is difficult. Each individual's DNA must be collected, tested, and the and processed to generate the relevant genomic data. Training the classification model requires training genomic data, where a previously determined classification for each individual is known (e.g., the type of cancer which the individual has been found to have). For less commonly occurring conditions, it may be difficult to collect enough genomic data training samples from individuals known to have that cancer. Where two cancers manifest similarly and are difficult to distinguish, it becomes increasingly likely that a large genomic training dataset will contain training samples with incorrectly pre-determined classifications (i.e. where an individual has been diagnosed with the first type of cancer, but in actuality has the other type of cancer) .

As a result, it is difficult to develop sufficiently accurate regression-based classification models using commercially available genomic datasets. The number of features in genomic data also makes statistical regression analysis computationally expensive, further limiting its practical applications.

Accordingly, there is a need to provide improved methods for utilising genomic datasets which allow classification models to be trained more easily and accurately.

### Summary of the Invention

According to a first embodiment, there is provided a method for generating a lower-dimensionality vector for training a classification model, the method comprising: receiving genomic data; pre-processing the genomic data to generate an initial vector of *M* elements, each element of the initial vector representing at least one gene expression; for each element in the initial vector, assigning at least one genetic identifier value from a known set of genetic identifier values to the element based on at least one genetic identifier associated with a gene expression of that element; performing dimensionality reduction on the initial vector by referencing the at least one genetic identifier value associated with each element of the initial vector, to generate a lower-dimensionality vector of *K* elements where *K* < *M;* and outputting the lower-dimensionality vector.

By performing dimensionality reduction with reference to the at least one associated genetic identifier value, the first embodiment enables classification the development of classification models which are more accurate, and at lower computational cost. The lower-dimensionality vector *K* can be made to contain elements associated with genetic identifiers of interest, allowing the dimensionality of the initial vector to be reduced while ensuring the most important features remain

Optionally, the method further comprises clustering the elements of the initial vector into clusters according to the genetic identifier value assigned to each element and generating a clustered vector comprising the clusters, optionally wherein the generation of the lower-dimensionality vector comprises selecting elements of a subset of the clusters from the clustered vector as elements of the lower-dimensionality vector, and further optionally wherein the clusters whose elements are selected for the lower-dimensionality vector are the clusters which have been determined to have the greatest importance for training the classification model. Clustering the elements according to their genetic identifier values may advantageously allow new correlations to be identified between a cancer and a particular genetic identifier.

Optionally, the generation of the lower-dimensionality vector comprises setting a parameter characterising the elements of a cluster from the clustered vector as an element of the lower-dimensionality vector, and optionally wherein the parameter is one of the maximum, minimum, mean, median, standard deviation, skewness, or kurtosis value of the elements in the cluster. Setting a parameter characterising the elements of the cluster advantageously allows a cluster to be represented by a smaller number of elements, reducing the size of the lower-dimensionality vector.

Optionally, the genetic identifier is the chromosome to which a gene expression represented by that element belongs, and the genetic identifier value is the chromosome number of that chromosome.

Optionally, a genetic identifier is the biological pathway to which a gene expression represented by that element belongs, and the genetic identifier value is a number identifying that biological pathway.

Optionally, a genetic identifier is the chromosome region to which the gene expression of that element belongs, and the genetic identifier value is a number identifying that region. Certain regions are known or thought be associated with oncogenes, and may therefore be of greater importance for classification.

Optionally, a first genetic identifier value and a second genetic identifier value are assigned to each element of the initial vector, and dimensionality reduction on the initial vector is performed by referencing the first and second genetic identifier values associated with each element of the initial vector, wherein the first and second genetic identifier values are associated with different types of genetic identifier of the gene expression of that element. This multidimensional approach may enable the identification of potential biomarkers by considering both the location and functional relevance of specific genetic factors associated with that cancer

According to a second embodiment, there is provided a method of classifying the cancer of a patient, comprising: processing a cancer cell sample from the patient, to obtain patient genomic data; generating a lower-dimensionality patient vector, the lower-dimensionality patient vector comprising the lower-dimensionality vector generated from the patient genomic data according to the dimensionality reduction method of any previous embodiment; and processing the lower-dimensionality patient vector using a classification model to classify the patient's cancer.

Optionally, the classification model is trained by: a. receiving genomic data for use as training data; b. generating lower-dimensionality training data, the lower-dimensionality training data comprising a plurality of lower-dimensionality vectors generated from the genomic training data according to the dimensionality reduction method; and c. training the classification model using the lower-dimensionality training data.

According to a third embodiment, there is provided a method for generating a two-dimensional representation for training a classification model , the method comprising the steps of: receiving genomic data; pre-processing the genomic data to generate an initial representation, each element of the initial representation comprising a gene expression; for each element in the initial representation, assigning at least one genetic identifier value from a known set of values to the element based on at least one genetic identifier associated with a gene expression of that element; generating a mapping matrix by referencing the at least one genetic identifier value associated with each element of the initial representation, operating the mapping matrix on the initial representation to generate a re-arranged representation; and outputting the re-arranged representation.

According to a fourth embodiment, there is provided a method of classifying the cancer of a patient, comprising: processing a cancer cell sample from the patient, to obtain patient genomic data; generating a re-arranged representation using the patient genomic data according to the re-arrangement method of any previous embodiment; and processing the re-arranged patient representation using a classification model to classify the patient's cancer.

Optionally, the method further comprises the step of clustering the elements of the initial representation into clusters according to the genetic identifier value assigned to each element, and wherein the mapping matrix operates on the initial representation by re-arranging the elements of each of the clusters of the initial representation as one of: a. a row of the re-arranged representation; b. a column of the re-arranged representation; c. a contiguous sub-representation within the re-arranged representation; and d. a rectangular contiguous sub-representation within the re-arranged representation.

Optionally, the method further comprises the step of clustering the elements of the initial representation into clusters according to the genetic identifier value assigned to each element, and wherein the mapping matrix operates on the initial representation by re-arranging the elements of each of the clusters of the initial representation so as to maximise the mixing of elements of different clusters within the re-arranged representation.

Optionally, the classification model is trained by: a. receiving genomic data for use as training data; b. generating a re-arranged training data representation, the re-arranged training data representation comprising a plurality of re-arranged training data representations generated from the genomic training data according to the re-arrangement method; and c. training the classification model using the re-arranged training data representation.

Optionally, the classification model is for classifying cancer types, optionally small cell lung cancer (SCLC) and non-small cell lung cancer (NSCLC), such as large cell lung cancer (LCLC), lung adenocarcinoma (AD) and squamous cell carcinoma (SCC) of the lung. Advantageously, experimental data has shown that such classification models are more effective than existing classification models used for these cancers.

### Brief Description of the Drawings

Figures 1(a) and (b) graphically illustrate a method for training a classification model as known in the art.
Figure 2 graphically illustrate a method for training a classification model according to an embodiment of the invention.
Figure 3 is a diagrammatic view of a chromosome illustrating the chromosome regions.
Figures 4(a) and (b) graphically illustrate how dimensionality reduction can be performed according to embodiments of the invention. Figures 4(c) and 4(d) are flowcharts setting out a method for performing dimensionality reduction.
Figure 5 graphically illustrates a method for training a classification model using a two-dimensional representation as known in the art.
Figure 6 graphically illustrates a method for training a classification model using a two-dimensional representation according to an embodiment of the invention.
Figures 7(a) to (c) illustrate how the elements in a 2D representation may be arranged according to various embodiments of the invention.

### Detailed Description

"Patient" and "subject" may be used interchangeably and each refers to the subject whose cancer is to be classified. In some examples, the patient is human.

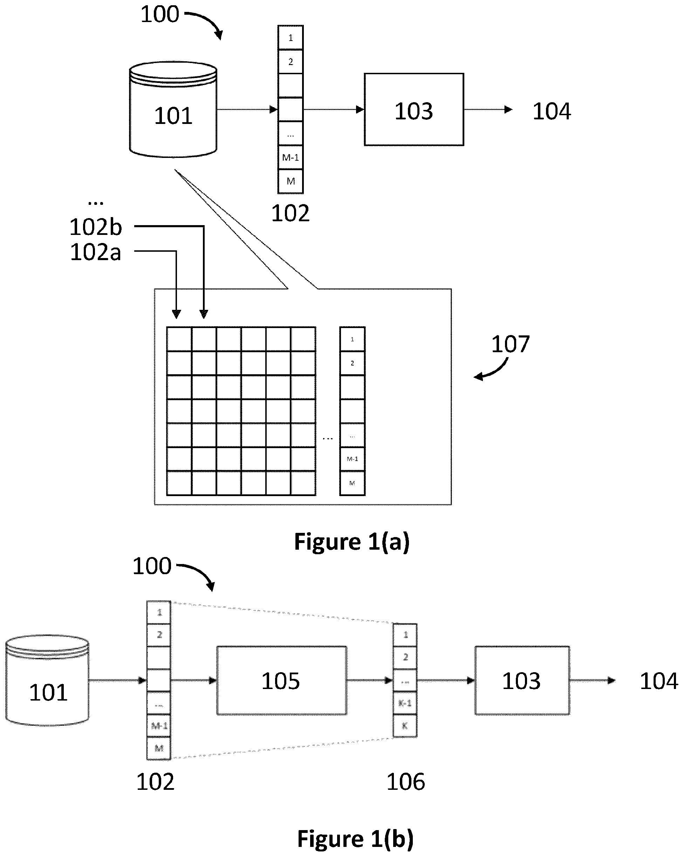

Turning to Figure 1(a), a simple method 100 for training a classification model which will first be described. First, the genomic data 101 is received. It will be understood that genomic data may include any way of numerically representing one or more individual's DNA or genome, such as their gene expressions.

The genomic data is pre-processed to generate an initial array 107 of *N* vectors 102, with each of the *M* rows corresponding to a feature (in other words, the array 107 is an *MxN* matrix of elements). Pre-processing the data may also include splitting the genomic data 101 into training and validation data, and other techniques known in the art may also be applied. The data in each vector 102 represents a distinct test sample of genomic data - in other words, each vector 102 represents the genomic data of an individual person. The element in each row 1-*M* of the array 107 represents at least one gene expression, in the sense that the element contains numerical information about the gene expression. A gene expression can be numerically represented by its gene expression level, which characterises the amount of protein which the gene is producing in a particular cell or tissue. The gene expression level may be a value between 0, meaning zero expression, and 1, meaning maximum expression. Alternatively, the gene expression level may be quantified as an absolute number, such as the amount of associated protein measured. The gene expression level may be taken as the average gene expression level across a number of cell or tissue samples. In an example, the numerical value of the element in row *i* of vectors 102a and 102b may be the average gene expression level of a particular gene *i* in the respective genetic data of two individuals A and B. The numerical value of an element in the initial vector may alternatively represent the gene expression level of a plurality of genes, such as the mean of their individual gene expression levels.

The array 107 is then used to train the classification model 103. The term "classification model" may refer to any model for performing classification known in the art. This may include machine learning models and convolutional neural network models, as well as simpler models based on linear regression. In the embodiment shown, the classification model is trained using a machine learning approach. Iterating sequentially through the columns 1-*N* of the array 107, each column is input as the initial vector 102 to the classification model 103. Alternatively, the classification model 103 could operate on each column of the array 107 in parallel using efficient vector mathematical operations.

For each input vector 102 (i.e. for each individual), the classification model 103 makes a classification prediction 104. The classification prediction 104 may include the probability that an individual has a particular type of cancer, and/or the probability that the individual will develop a particular type of cancer. The model is trained using genomic training data 101, in which a pre-determined classification for each individual is known (e.g., the type of cancer which the individual has been found to have). By comparing the classification prediction 104 to the true classification, the parameters of the classification model 103 can be trained to make more accurate classification predictions 103 about the training data.

However, the method as described and in Figure 1(a) will perform poorly due to the previously discussed "curse of dimensionality". Since the length (also known as the dimensionality) of the input vector *M* is large compared to the number of training samples *N,* the accuracy of the correlation model 103 will be limited. The correlation model 103 will also become increasingly computationally intensive to calculate as the number of features *M* increases.

An alternate method 200 for training a classification model is now described with reference to Figure 1(b). After pre-processing the genomic data 101 to generate the array 107, the initial array 107 is passed to a dimensionality reduction stage 105. For each initial vector 102 in the initial array 107, the dimensionality reduction stage 105 generates a lower-dimensionality vector 106 of *K* elements where *K* < *M.* An example of a simple way to perform dimensionality reduction is simply to select the first K elements of each input vector 102 as the elements of the lower-dimensionality vector 106. The lower-dimensionality vectors 106 are then passed to the classification model 103 (either sequentially or as a single array as previously discussed) to train the model. Since *K* < *M,* the dimensionality complexity is reduced. However, by reducing the number of features, crucial information for training the classification model 103 may be lost.

It is known in the art to use data-driven algorithms (such as machine learning methods) for the dimensionality reduction stage 105, in order to reduce the dimensionality M of the input vector 102 by projecting it to a lower-dimensionality vector of dimensionality K < M. Although such dimensionality reduction methods improve the performance of the classification model 103, again they rely on training from the available *N* samples in the genomic training data 101, which are usually not enough. Thus, such algorithms are also subject to the same previously described dimensionality problems, and cannot offer significant performance improvements. Due to the complex mathematical nature of machine-learning, it can be difficult to determine why particular features were selected. This limits the usefulness of such algorithms, particularly in understanding the underlying causal genomic factors.

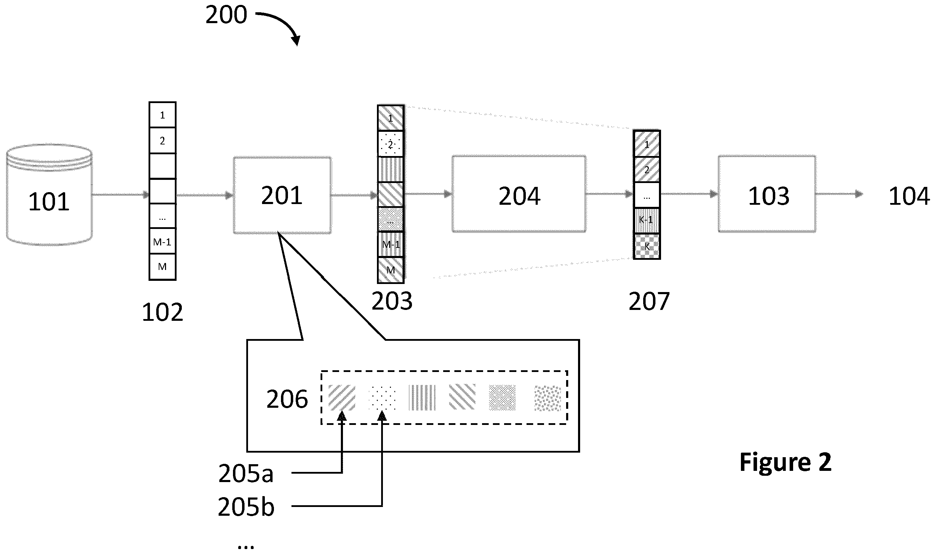

Turning to Figure 2, a method 300 for training a classification model 103 according to an embodiment of the invention is shown. Rather than using a purely data-driven algorithm for the dimensionality reduction stage 105, this method uses a knowledge-based approach. Specifically, knowledge about genetic identifiers associated with particular gene expressions is used to improve the dimensionality reduction stage 105, and thereby improve the training of the classification model 103.

After pre-processing the genomic data 101 and generating the initial array 107 of initial vectors 102, each vector 102 (or the initial array 107 in its entirety) is passed to a genetic identifier assignment stage 201. This stage 201 assigns a genetic identifier value 205a, 205b, ..., from a known set 206 of genetic identifier values 205 to the element based on a genetic identifier associated with a gene expression of that element. Once assigned, the genetic identifier assignment stage 201 outputs an assigned vector 203.

For clarity, the different genetic identifier values 205 are each represented graphically as patterns. Thus, in the assigned vector 203, the patterns of the elements *1-M* illustrate that each element has been assigned a genetic identifier value 205 based on a genetic identifier associated with a gene expression of that element.

The term "genetic identifier", when associated with a gene expression of an element, refers to known information which can identify or locate the particular gene expression. One genetic identifier may be the chromosome on which the corresponding gene expression is located. If the location of the gene on one of the 22 human chromosome pairs is known (note that there are 23 chromosome pairs, but one of these pairs will be the sex chromosomes X and Y), the genetic identifier value 205 can be set as a number between 1 and 22. If the element represents a plurality of gene expressions, then the genetic identifier value 205 may be the chromosome pair in which all the gene expressions are located, on which most of the gene expressions are located, or on which a particular gene expression or expressions is located.

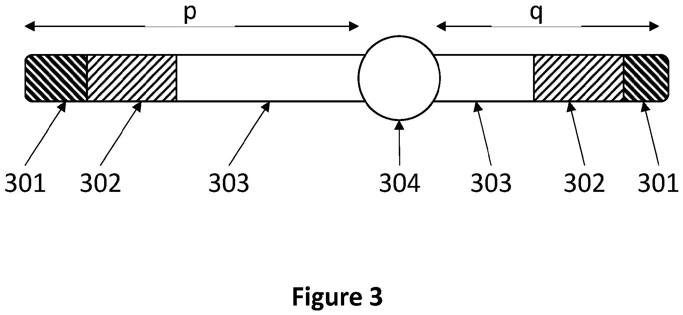

Alternatively, or additionally, the genetic identifier associated with a gene expression may be the chromosome region where that gene expression is located. There are several conventionally identified regions on each chromosome. As shown by Figure 3, chromosome regions may be defined as include the telomere 301 end regions, sub-telomere regions 302 bordering the telomeres, pericentric regions 303 towards the centre of the chromosome, and the centromere region 304. Where the chromosome is submetacentric (having a larger and smaller arm, as shown in Figure 3), the larger arm forms the "q" region and the smaller arm the "p" region of the chromosome. In this case, the genetic identifier value would be a number identifying the chromosome region where the gene expression is located.

Chromosome regions provide several advantages as a genetic identifier. Specific chromosome regions are known or theorised to contain oncogenes (mutated genes with a higher potential to cause cancer), or conversely tumour suppressor genes which reduce a risk for cancer. When performing dimensionality reduction, selecting gene expressions associated with such chromosome regions may provide improved prediction modelling and risk assessment, as well as monitoring/predicting the malignancy of an existing cancer. Some chromosome regions are particularly associated with certain types of cancer, such as lung cancer. When developing diagnostic tests and therapies for e.g. lung cancer, it may be advantageous to select gene expressions corresponding with such associated chromosome regions. Early detection of such cancers can also be improved by utilising these regions.

In a further alternative, the genetic identifier associated a gene expression may be a biological pathway associated with that gene expression. Biological pathways include metabolic, genetic and signal transduction pathways. There are approximately 2000 known biological pathways. In this case, the genetic identifier value 205 would be a number identifying the biological pathway. The known set 206 of genetic identifier values 205 may be defined by a standard convention or database, such as the Reactome online database.

The genetic identifier assignment stage 201 may assign multiple genetic identifier values 205 to an element of an initial vector 102. For example, the genetic identifier value may assign a combination of the chromosome number, chromosome region number, and genetic pathway number to an element. Where an element contains information about multiple gene expressions, the genetic identifier assignment stage 201 may assign genetic identifier values for each gene expression in the element. For example, the chromosome numbers for all the gene expressions represented by the element may be assigned.

The genetic identifier value 205 for each gene expression may be included in the received genomic data 101. For example, each gene expression datapoint may have an associated gene ID which includes the chromosome number, chromosome region and/or genetic pathway.

The assigned vector 203 is then passed to a knowledge-based dimensionality reduction stage 204. It should be understood that the genetic identifier values 205 may be stored in a separate array or assigned vector 203, which can then be passed to the dimensionality reduction stage along with the initial vector 102. The dimensionality reduction stage 105 is performed by reference to the genetic identifier value 205 associated with each element of the initial vector 102. The dimensionality reduction stage 204 outputs a lower dimensionality vector 207 of *K* elements where *K* < *M.*

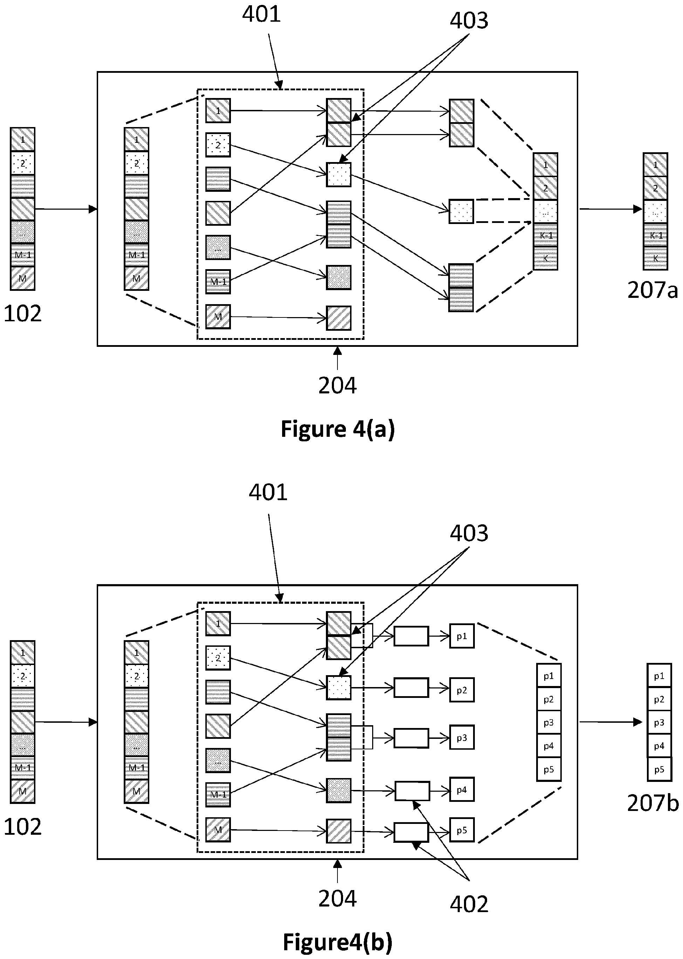

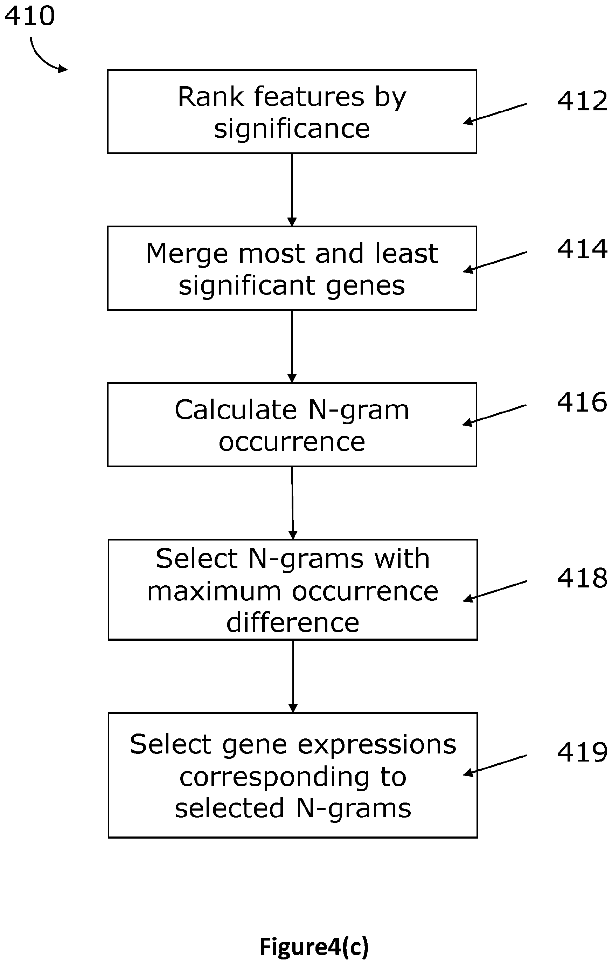

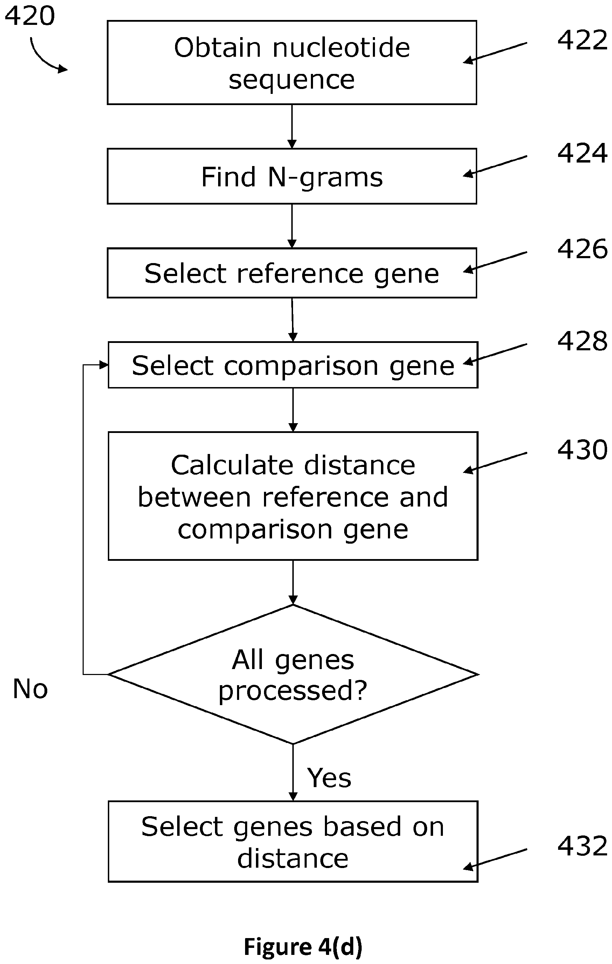

Referring to Figure 4, the dimensionality reduction stage 204 may include the steps of clustering 401 the elements of the initial vector 102 based on the genetic identifier value 205 associated with each element of the initial vector 102. In the embodiment shown, the elements are clustered so that each cluster 403 contains only elements associated with a sub-set of the set 206 of genetic identifier values 205. For example, the first cluster 403 could only contain elements representing gene expressions located on chromosome 1 or representing gene expressions associated with a particular biological pathway. Alternatively (not shown), the elements may be clustered so as to maximise the mixing of different genetic identifier values 205 within each cluster 403. Once sorted into clusters, the elements may be arranged to as a clustered vector in which the elements of each cluster 403 are contiguous. For example, the clustered vector could list all of the elements representing a gene expression located on chromosome 1, followed by all the elements representing a gene expression located on chromosome 2, continuing until the clustered vector contains lists all of the clusters of interest.

In a further alternative, a multidimensional approach may be used in which elements are clustered using a combination of genetic identifiers. The multidimensional approach contributes to the development of a more accurate predictive model, and is particularly relevant for cancer types where multiple genetic factors are known or thought to contribute to the pathology.

In such a multidimensional approach, a first genetic identifier value and a second genetic identifier value may be assigned to each element of the initial vector, and dimensionality reduction on the initial vector may be performed by referencing the first and second genetic identifier values associated with each element of the initial vector, wherein the first and second genetic identifier values are associated with different types of genetic identifier of the gene expression of that element.

For example, elements may be clustered based on a combination of their associated chromosome number (serving as the first genetic identifier) and genomic pathway (serving as the second genetic identifier). By incorporating spatial genomic information (chromosome regions) and functional genomic information (pathways), this approach may enable the identification of potential biomarkers by considering both the location and functional relevance of specific genetic factors associated with that cancer. The combination of chromosome region and pathway data facilitates the identification of regulatory networks governing gene expression, which may help to identify the intricate control mechanisms influencing cellular functions.

In one embodiment, a subset of the clusters 403 is selected for the lower-dimensionality vector 207b, as shown in Figure 4(a). For example, one may select only the clusters 403 containing elements representing the gene expressions located on chromosomes 1, 2 and 3, and discard the clusters 403 containing elements representing gene expressions located on other chromosomes. This may be advantageous when a cancer of interest is known to have a genetic link with gene expressions on a particular chromosome or chromosomes. By selecting the clusters 403 representing the chromosomes of interest, the dimensionality of the initial vector 102 can be reduced while ensuring the most important features remain. That is, the clusters selected are the clusters which have been found to comprise the most significant features for accurately training a classification model to perform a particular classification. To further reduce the dimensionality of the lower-dimensionality vector 207b, a subset of the clusters 403 may first be selected, then a subset of the elements of each of those clusters 403 selected for the lower-dimensionality vector 207b.

Clustering the elements according to their genetic identifier values 205 may also allow new correlations to be identified between a cancer and a particular genetic identifier. A low-dimensionality vector is generated by selecting a cluster containing only elements associated with a first genetic identifier value 205. A first classification model 103 is trained using the low-dimensionality, and the accuracy of the classification model at identifying that cancer is tested. Classification models are then trained and tested using low-dimensionality vectors containing elements associated with each subsequent genetic identifier value 205 in the set of genetic identifier values 206. If a classification model has a high accuracy when trained on data associated with a particular genetic identifier value 205, then it is likely there is a correlation between that cancer and the genetic identifier value. For example, if a particular cancer can be accurately identified using elements representing gene expressions located on chromosome 1, but cannot be accurately identified when using elements representing gene expressions located on chromosome 2, this can indicate that there may be a genetic link between the identified cancer and the gene expressions of chromosome 1.

As shown in Figure 4(b), the dimensionality can be alternatively, or additionally, reduced by calculating 402 parameters *p* representing each cluster 403. The dimensionality of each cluster having u elements is reduced by calculating a number *p <* u parameters to represent the cluster. Parameters *p₁* are calculated for the first cluster 401a, *p₂* for the second cluster 401b, and so on. The parameters *p* calculated for each cluster 403 are set as the elements of the lower-dimensionality vector 106. The parameters *p* calculated for each cluster may include the mean, median, standard deviation, skewness, or kurtosis value of the elements within that cluster 403. For example, where the elements in the cluster 403 each represent the gene expression level of a unique gene expression, the mean of the elements will represent the average gene expression level of the gene expressions in the cluster 403. In one embodiment, the set of parameters *p* is the same for each cluster 403. Alternatively, different parameters may be calculated depending on the size and characteristics of each cluster 403.

In all cases, the same dimensionality reduction is applied to all initial vectors 102 in the initial array 107.

Figure 4(c) illustrates steps of a subsequence occurrence-based method 410 which may be performed as part of a dimensionality reduction method as previously described.

In step 412, feature ranking is performed to find the A most significant and the A least significant gene expressions from a set of genomics data. Any feature ranking method known in the art may be used. The most significant gene expressions may be determined by reference to the associated genetic identifier values. For example, the A most significant gene expressions may be those belonging to a particular chromosome region, and the A least significant gene expressions may be those belonging to chromosome regions furthest away from the first chromosome region, or on an entirely different chromosome.

In step 414, the A most significant and A least significant gene expressions are merged, and the B most frequently occurring nucleotide sequences of length N (nucleotide sequences of length N will be referred herein as "N-grams") are found and selected. Merging the gene expressions may comprise grouping, selecting and concatenating the gene expressions into one or more subgroups.

In step 416, the average number of occurrences of each N-gram in the best A genes and in the A worst genes is calculated. This may involve counting how many times each of the subsequences appear in each gene expression.

In step 418, the D N-Grams with the maximum average occurrence difference between the A most significant and the A less significant gene expressions are found and selected from the B most frequently occurring N-grams.

In step 419, the S gene expressions containing or being associated with the D N-grams at least P times are selected. The elements of the initial vector associated with these S gene expressions are then selected for the lower-dimensionality vector.

Figure 4(d) illustrates steps of another method 420 which may be performed as part of a dimensionality reduction method as previously described. This method 420 uses region-based feature selection followed by sequence alignment clustering.

In step 422, a nucleotide sequence is obtained for each gene expression from a set of genomics data. In some examples, this comprises obtaining a nucleotide sequence corresponding to the gene expression represented by each element of the initial vector.

In step 424, the N-grams for the nucleotide sequence are found.

In step 426, a reference gene expression is selected. The reference gene may be selected as a gene whose expression is known to correlate to a particular cancer, for example. To reduce the computational complexity in some examples, only genes with specific and desired correlation to cancer are selected as reference ones.

In step 428, another gene expression (the comparison gene expression) is selected from the genomics dataset or initial vector.

In step 430, the distance between the reference and comparison gene expressions is calculated. This may be done by calculating the Euclidian distance between the reference gene expression and the comparison gene expression. Steps 428-430 are repeated for all gene expressions in the genomics dataset.

In step 432, S gene expressions are then selected based on their distance to the reference gene expression. For example, K-means clustering may be used to find the M gene expressions which are closest to the reference gene.

In a further embodiment, the knowledge-based training method described above is adapted for use with convolutional neural networks (CNNs). Convolutional neural networks are a sub-category of machine learning methods. Whereas other machine learning methods take a 1-dimensional vector as input, the input to CNNs is commonly a 2-dimensional representation (for example, an image). Similarly, whereas the training of classification models based on other machine learning methods is improved by reducing the dimensionality of the initial vector, the training of classification models based on CNNS is improved by re-arranging the initial 2D representation.

A 1-D vector and a 2-D representation can each be transformed by re-arranging the order of their respective elements. However, unlike a 1-D vector, a 2-D representation can also be transformed by changing the shape of the representation. For example, a 10 x 10 element 2D representation can be transformed into a 20 x 5 element 2D representation with no loss of information. A 1D vector can also be re-arranged into a 2D representation, and vice versa. For example, a vector with 100 elements can be arranged into a 10 x 10 element 2D representation.

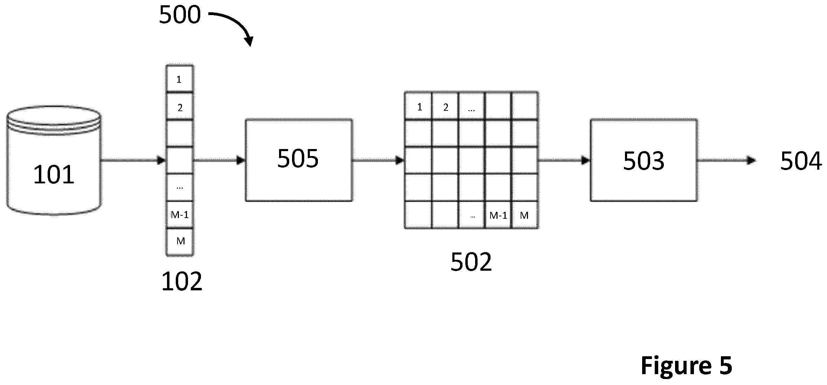

Referring now to Figure 5, a method 500 for training a classification model using a CNN is shown. As with previously described methods, genomic data 101 is received and pre-processed to form an array 107 of initial vectors 102. The initial vectors 102 are then passed to a re-arrangement stage 505, where they are re-arranged from a *1 x M* vector comprising *M* elements to an *A x B* (i.e., *A* rows with *B* columns in each row) 2D representation 502 still comprising *M* elements. Once re-arranged, the 2D representation 502 is passed to the classification model 503, where it is used to train the classification model 503 to make classification predictions 504.

One method for re-arranging the initial vector 102 into a 2D representation 502 is to arrange the elements sequentially. For example, the first *B* elements of the initial vector 102 become the first row of the 2D representation 502, the second *B* elements become the second row, and so on. Another known method is to arrange the elements of the initial vector 102 into the 2D representation in a random order. In all cases, the same re-arrangement is applied to all initial vectors 102 in the initial array 107. As with previously described examples, the method will be discussed in relation to a single initial vector 102 of the initial array 107. In alternate embodiments, the method may be performed simultaneously or sequentially on multiple initial vectors 102 of the initial array 107.

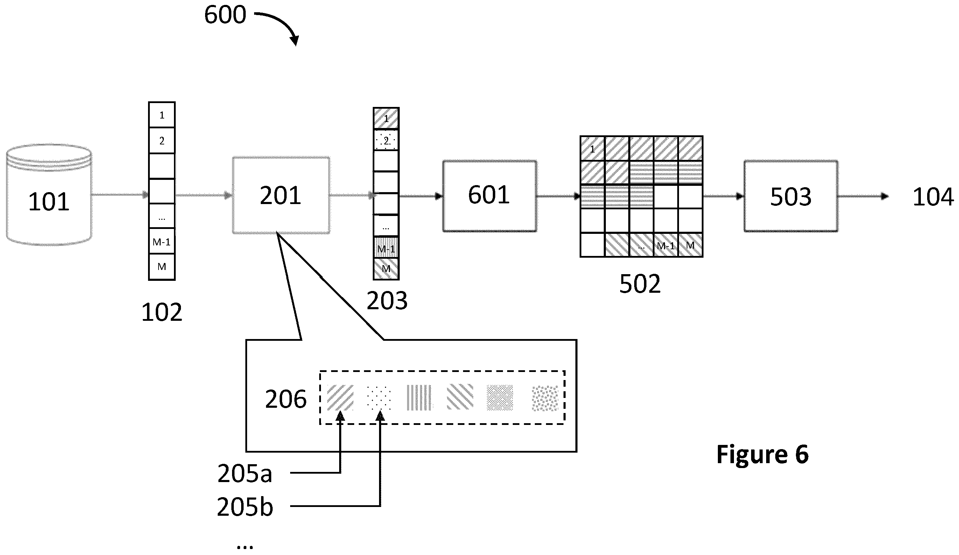

Turning to Figure 6, a method 600 is shown for training a classification model 503 is shown according to an embodiment of the invention. As previously described in relation to Figure 2, the genomic data 101 is received and pre-processed. The initial vector 102 is then passed to the genetic identifier assignment stage 201. As previously described, in this step a genetic identifier value 205a, 205b, ..., from a known set 206 of genetic identifier values 205 is assigned to the element based on a genetic identifier associated with a gene expression of that element. The assigned vector 203 is then output.

The assigned vector 203 is passed to a knowledge-based re-arrangement stage 601. In some examples, the first step of the re-arrangement stage 601 may be to perform a simple initial re-arrangement on the initial vector 102 to transform it into a 2D initial representation (for example, by arranging the elements sequentially as in the re-arrangement stage 505 of Figure 5). The simple first re-arrangement may improve the efficiency of the overall re-arrangement stage 601. This is because in some instances it can be computationally efficient to use a simple method to transform a 1D vector to a 2D representation and then carry out a complex operation to transform the 2D representation into a second 2D representation. A more computationally expensive option would be transforming the 1D vector directly into the second 2D representation. Alternatively, the assigned vector 102 may be treated as the initial representation without any initial re-arrangement.

In another example, the initial-rearrangement step and the genetic identifier assignment step 201 may take place in the opposite order, such that genetic identifier values are assigned to the rearranged initial representation rather than to the initial vector 102.

To perform the re-arrangement, the knowledge-based re-arrangement stage 601 is used to generate a mapping matrix by referencing the genetic identifier value 205 associated with each element of the initial representation. The mapping matrix is generated such that when it is applied to the initial representation 102, the initial representation 102 is transformed to generate a desired re-arranged representation 502.

As previously described in relation to the dimensionality reduction stage 204, the re-arrangement stage 601 may include the steps of clustering 401 the elements of the initial representation based on the genetic identifier value 205 associated with each element of the initial vector 102. The clusters may then themselves be re-arranged into a particular 2D representation within the re-arranged representation 502.

The re-arranged representation may then be passed to the classification model 503 and used to train the classification model 503.

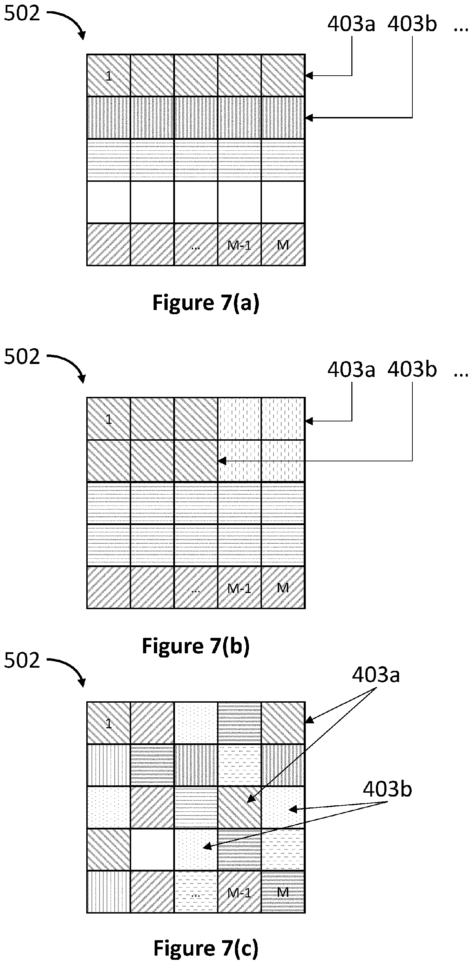

Figures 7(a) to (c) shows examples of possible ways in which the initial representation may be re-arranged by referencing the genetic identifier value 205 associated with each element of the initial representation, according to embodiments of the invention. In Figure 7(a), the clusters 403 of elements are arranged so that each cluster 403a, 403b, ..., forms a row of the re-arranged representation 502. The clusters 403 may be arranged to form the columns of the re-arranged matrix. In Figure 7(b), the clusters 403 of elements are arranged so that each cluster 403a, 403b, ..., forms a contiguous area of the re-arranged representation 502. The contiguous areas may be rectangular as shown, or they may be other shapes. In Figure 7(c), the clusters 403 are arranged so as to maximise mixing of each cluster 403a, 403b, ..., within the re-arranged representation 502. This arrangement can provide better and more uniformly distributed mixing of elements with different associated genetic identifier values 205 compared to purely random arrangement. This is because any large random arrangement is likely to have smaller areas where elements associated with particular genetic identifier values 205 are more or less concentrated.

A method according to an embodiment of the invention for training a classification model 103, 503 and using it to classify cancer is described below. The classification model 103, 503 is trained as previously described. Genomic training data 101 is received and is pre-processed to form an initial array 107 of initial vectors 102. The initial vectors 102 are then passed through a genetic identifier assignment stage 201 as previously described.

The initial vectors 102 and assigned vectors 203 of the genomic training data 101 are then processed, as previously described, to generate lower-dimensionality vectors 207. This is performed according to the knowledge-based dimensionality reduction stage 204, or rearranged into representations 502 according to the knowledge-based rearrangement stage 601. The lower-dimensionality or re-arranged genomic training data is then used to train a classification model 103, 503. Rather than using all of the genomic training data 101 to train the classification model 103, 503, a portion of the genomic training data 101 may be selected and used to validate the trained classification model 103, 503 using one of a variety of sampling techniques known in the art, such as random sampling.

A data sample of a patient can then be processed for use with the trained classification model 103, 503. A cell sample is obtained from a patient, which is then processed to generate a sample of genomic data pertaining to that patient. The patient genomic data is then successively passed through the same pre-processing, genetic identifier assignment stage 201, and dimensionality reduction 204 or re-arrangement 601 stage as were used for the genomic training data, outputting respectively a lower-dimensionality vector 106 or a re-arranged representation 502 of data pertaining to the patient. The genomic training data 101 and patient genomic data are processed in the same way, such that the patient genomic data is compatible with and suitable for the trained classification model 103, 503. The lower-dimensionality vector 106 or re-arranged representation 502 of the patient genomic data is then input into the trained classification model 103, 503, which then outputs a classification prediction 104.

The classification prediction 104 may be a prediction, based on the patient's genomics, of the likelihood of the patient developing a particular type of cancer or cancers. Appropriate action can then be taken to monitor the patient or mitigate the risk. For example, the patient may in future be screened regularly for the identified cancer type(s). The patient might also be advised to reduce potentially cancer-inducing activities, such as smoking. Alternatively, or additionally, the classification prediction 104 may be a diagnosis score based on genomic data sampled from tissue in a cancerous growth, indicating which type of cancer the growth signifies. Based on this, the patient can be prescribed the most appropriate treatment pathway for the identified cancer type. In some examples, the patient may be assigned to a particular cohort of patients for clinical trials, in order to help develop or test new treatment pathways for that cancer.

In some embodiments of the second aspect of the invention, the method further comprises the pre-step of obtaining a cancer cell sample from the patient.

Suitably, the cancer cell sample may be obtained from by a biopsy, blood sample, or other cell collection method. The patient's genomic data may be obtained from the cancer cell sample by methods known to the skilled person, such as genomic sequencing.

Step a. of the method of the second aspect of the invention (i.e. the step of processing a cancer cell sample from the patient, to obtain patient genomic data) may be carried out by a third party to the party performing steps b. and c.; therefore, in some embodiments of the second aspect of the invention, step a. is optional and step b is generating a re-arranged representation using patient genomic data obtained from processing a cancer cell sample.

In some examples, the classification model may be trained by:
a. receiving genomic data for use as training data;
b. generating lower-dimensionality training data, the lower-dimensionality training data comprising a plurality of lower-dimensionality vectors generated from the genomic training data according to the dimensionality reduction method; and
c. training the classification model using the lower-dimensionality training data.

The fourth aspect of the invention may have any of the features of the second aspect of the invention.

### Experimental Results

Classification models were trained using lower-dimensionality training datasets, the datasets each comprising a plurality of lower-dimensionality vectors generated from the genomic training data according to an exemplary dimensionality reduction method described above. The performance of the classification methods were then experimentally tested against other methods, with the results shown in the tables below. The following classification methods were experimentally tested.
a. Baseline method: no dimensionality reduction. In the baseline method, the output feature vector consists of all available gene expression values. This 'long' feature vector is used as input to a ML model for classification or regression.
b. Data-driven model: baseline method, but using known data-driven ML methods for dimensionality reduction
c. Region-based model: baseline method, but with the gene expressions clustered in groups based on their regions. The average importance of each region is estimated using a feature ranking algorithm. The most important regions are selected for the lower-dimensionality output vector.
d. Region-based feature selection followed by sequence alignment clustering: the region-based method, but with dimensionality reduction performed using the region-based feature selection followed by sequence alignment clustering method described in Figure 4(d)
e. Subsequence occurrence: dimensionality reduction performed according to the subsequence occurrence method described in Figure 4(c) using 50 N-grams, and best/worst 100 gene features (i.e. K = 50, M = 100).

In the first experimental test, the models were used to predict the immunotherapy result of kidney cancer, and melanoma on patients:

### Experiment 1: Immunotherapy Response Prediction

**Table 1: Summary of datasets:**

| | **Number of samples response** | **Number of samples non-response** | **Number of samples in total** | **Number of gene features** | **Clinical information available** |
|---|---|---|---|---|---|
| Kidney Cancer Immunotherapy Response Prediction | 64 | 165 | 229 | 15608 | No |
| Melanoma Immunotherapy Response Prediction | 124 | 162 | 286 | 12880 | No |

**Table 2: Results**

| **Prediction Type** | **Methods** | **F1 score** | **Accuracy** | **Precision** | **Recall** |
|---|---|---|---|---|---|
| Kidney Cancer Immunothera py Response Prediction | Baseline | 46.19 | 64.86 | 53.18 | 50.46 |
| | Data Driven | 58.61 | 69.68 | 62.83 | 59.54 |
| | Region based | 61.85 | 67.55 | 63.77 | 65.52 |
| | region-based feature selection followed by sequence alignment clustering | 58.03 | 64.45 | 59.46 | 58.36 |
| | **Subsequence occurrence** | **68.82** | **69.30** | **68.43** | **69.30** |
| Melanoma Immunothera py Response Prediction | Baseline | 64.32 | 66.48 | 67.08 | 64.67 |
| | Data Driven | 66.08 | 66.79 | 66.75 | 66.62 |
| | Region based | 66.44 | 67.86 | 67.61 | 66.62 |
| | **region-based feature selection followed by sequence alignment clustering** | **71.52** | **73.13** | **72.51** | **72.15** |
| | Subsequence occurrence | 63.31 | 63.07 | 66.67 | 63.07 |

As Table 1 shows, the Subsequence occurrence method provides the best accuracy, prediction and recall for kidney cancer prediction, but the region-based feature selection followed by sequence alignment clustering method performs best for melanoma prediction.

In a second experimental test, the models were used to predict for recurrence of lung cancer in patients:

### Experiment 2: Recurrence Prediction

**Table 3: Summary of Dataset**

| | **Number of samples response** | **Number of samples non-response** | **Number of samples in total** | **Number of gene features** | **Clinical information available** |
|---|---|---|---|---|---|
| Kidney Cancer Immunotherapy Response Prediction | 88 | 269 | 357 | 4757 | pathologic M, pathologic N, pathologic T, sex, history of neoadj uva nt treatment, age, Cancer type |

**Table 4: Results**

| **Prediction Type** | **Methods** | **F1 score** | **Accuracy** | **Precision** | **Recall** |
|---|---|---|---|---|---|
| Lung cancer Recurrence prediction | Baseline | 62.38 | 68.96 | 68.96 | 58.08 |
| | Data Driven | 67.83 | 70.63 | 70.63 | 66.11 |
| | Region based | 72.72 | 72.57 | 72.57 | 73.73 |
| | **region-based feature selection followed by sequence alignment clustering** | **74.25** | **74.25** | **73.56** | **75.34** |
| | Subsequence occurrence | 68.33 | 68.10 | 68.10 | 69.24 |

Table 2 shows that the region-based feature selection followed by sequence alignment clustering method performs best for a lung cancer recurrence prediction.

### Examples according to the invention

The following clauses are examples according to the invention:
1. A method for generating a lower-dimensionality vector for training a classification model, the method comprising:
   receiving genomic data;
   pre-processing the genomic data to generate an initial vector of *M* elements, each element of the initial vector representing at least one gene expression;
   for each element in the initial vector, assigning at least one genetic identifier value from a known set of genetic identifier values to the element based on a genetic identifier associated with a gene expression of that element;
   performing dimensionality reduction on the initial vector by referencing the at least one genetic identifier value associated with each element of the initial vector, to generate a lower-dimensionality vector of *K* elements where *K* < *M;* and
   outputting the lower-dimensionality vector.
2. The method of clause 1, wherein the method further comprises clustering the elements of the initial vector into clusters according to the genetic identifier value assigned to each element and generating a clustered vector comprising the clusters.
3. The method of clause 2, wherein the generation of the lower-dimensionality vector comprises selecting elements of a subset of the clusters from the clustered vector as elements of the lower-dimensionality vector.
4. The method of claim 3, wherein the clusters whose elements are selected for the lower-dimensionality vector are the clusters which have been determined to have the greatest importance for training the classification model.
5. The method of any of clauses 2 to 4, wherein the generation of the lower-dimensionality vector comprises setting a parameter characterising the elements of a cluster from the clustered vector as an element of the lower-dimensionality vector
6. The method of any of clause 5, wherein the parameter is one of the maximum, minimum, mean, median, standard deviation, skewness, or kurtosis value of the elements in the cluster.
7. The method of any preceding clause, wherein the genetic identifier is the chromosome to which a gene expression represented by that element belongs, and the genetic identifier value is the chromosome number of that chromosome.
8. The method of any preceding clause, wherein a genetic identifier is the biological pathway to which a gene expression represented by that element belongs, and the genetic identifier value is a number identifying that biological pathway.
9. The method of any preceding clause, wherein a genetic identifier is the chromosome region to which the gene expression of that element belongs, and the genetic identifier value is a number identifying that region.
10. The method of any of clauses 2-9, wherein the genetic identifier values of the elements within a cluster form a smaller subset of the set of genetic identifier values found within the initial vector.
11. The method of clause 10, wherein all of the elements within a cluster share the same genetic identifier value.
12. The method of any preceding clause, wherein each element of the initial vector comprises a unique combination of gene expressions from the plurality of gene expressions.
13. The method of any preceding clause, wherein
   a first genetic identifier value and a second genetic identifier value are assigned to each element of the initial vector, and
   dimensionality reduction on the initial vector is performed by referencing the first and second genetic identifier values associated with each element of the initial vector,
   wherein the first and second genetic identifier values are associated with different types of genetic identifier of the gene expression of that element.
14. The method of any preceding clause, wherein performing dimensionality reduction comprises the steps of:
   selecting the A most and A least significant gene expressions within the genomic data;
   merging the A most and least significant gene expressions;
   selecting the B most frequently occurring N-grams within the merged gene expressions; and
   selecting, from the B N-grams, the D N-grams with the maximum average occurrence difference; and
   selecting, from the D N-grams, the S elements of the initial vector representing gene expressions containing or being associated with the D N-grams at least P times.
15. The method of any preceding clause, wherein performing dimensionality reduction comprises the steps of:
   obtaining a nucleotide sequence corresponding to the gene expression represented by each element of the initial vectors;
   processing the nucleotide sequences to find corresponding N-grams
   selecting a reference gene expression from the genomics data;
   for each element of the initial vector, calculating the distance between the gene expression represented by the element and the reference gene expression; and
   selecting the S gene expressions for the lower-dimensionality vector based on their distance to the reference gene expression.
16. A method of classifying the cancer of a patient, comprising:
   a. processing a cancer cell sample from the patient to obtain patient genomic data;
   b. generating a lower-dimensionality patient vector, the lower-dimensionality patient vector comprising the lower-dimensionality vector generated from the patient genomic data according to the dimensionality reduction method of any previous clause; and
   c. processing the lower-dimensionality patient vector using a classification model to classify the patient's cancer.
17. The method of clause 16, wherein the classification model is trained by:
   a. receiving genomic data for use as training data;
   b. generating lower-dimensionality training data, the lower-dimensionality training data comprising a plurality of lower-dimensionality vectors generated from the genomic training data according to the dimensionality reduction method; and
   c. training the classification model using the lower-dimensionality training data.
18. A method for generating a two-dimensional representation for training a classification model, the method comprising the steps of:
   receiving genomic data;
   pre-processing the genomic data to generate an initial representation, each element of the initial representation comprising a gene expression;
   for each element in the initial representation, assigning a genetic identifier value from a known set of values to the element based on a genetic identifier associated with a gene expression of that element;
   generating a mapping matrix by referencing the genetic identifier value associated with each element of the initial representation,
   operating the mapping matrix on the initial representation to generate a re-arranged representation; and
   outputting the re-arranged representation.
19. The method of clause 18, further comprising the step of clustering the elements of the initial representation into clusters according to the genetic identifier value assigned to each element, and
   wherein the mapping matrix operates on the initial representation by re-arranging the elements of each of the clusters of the initial representation as one of:
   a. a row of the re-arranged representation;
   b. a column of the re-arranged representation;
   c. a contiguous sub-representation within the re-arranged representation; and
   d. a rectangular contiguous sub-representation within the re-arranged representation.
20. The method of clause 18 or 19, further comprising the step of clustering the elements of the initial representation into clusters according to the genetic identifier value assigned to each element, and wherein the mapping matrix operates on the initial representation by re-arranging the elements of each of the clusters of the initial representation so as to maximise the mixing of elements of different clusters within the re-arranged representation.
21. A method of classifying the cancer of a patient, comprising:
   a. processing a cancer cell sample from the patient to obtain patient genomic data;
   b. generating a re-arranged representation using the patient genomic data according to the re-arrangement method of any of clauses 12 to 14; and
   c. processing the re-arranged patient representation using a classification model to classify the patient's cancer.
22. The method of clause 21, wherein the classification model is trained by:
   a. receiving genomic data for use as training data;
   b. generating a re-arranged training data representation, the re-arranged training data representation comprising a plurality of re-arranged training data representations generated from the genomic training data according to the re-arrangement method; and
   c. training the classification model using the re-arranged training data representation.
23. The method of any preceding clause, wherein the classification model is for classifying a cancer type, preferably the cancer type is selected from the list consisting of small cell lung cancer (SCLC) and non-small cell lung cancer (NSCLC), such as large cell lung cancer (LCLC), lung adenocarcinoma (AD) and squamous cell carcinoma (SCC) of the lung.
24. The method of any preceding clause, wherein the classification model is for predicting cancer re-occurrence.

## Claims

1. A method for generating a lower-dimensionality vector for training a classification model, the method comprising:
receiving genomic data;
pre-processing the genomic data to generate an initial vector of *M* elements, each element of the initial vector representing at least one gene expression;
for each element in the initial vector, assigning at least one genetic identifier value from a known set of genetic identifier values to the element based on at least one genetic identifier associated with a gene expression of that element;
performing dimensionality reduction on the initial vector by referencing the at least one genetic identifier value associated with each element of the initial vector, to generate a lower-dimensionality vector of *K* elements where *K* < *M;* and
outputting the lower-dimensionality vector.

2. The method of claim 1, wherein the method further comprises clustering the elements of the initial vector into clusters according to the genetic identifier value assigned to each element and generating a clustered vector comprising the clusters, optionally
wherein the generation of the lower-dimensionality vector comprises selecting elements of a subset of the clusters from the clustered vector as elements of the lower-dimensionality vector, and further optionally
wherein the clusters whose elements are selected for the lower-dimensionality vector are the clusters which have been determined to have the greatest importance for training the classification model.

3. The method of claim 2, wherein the generation of the lower-dimensionality vector comprises setting a parameter characterising the elements of a cluster from the clustered vector as an element of the lower-dimensionality vector, and optionally
wherein the parameter is one of the maximum, minimum, mean, median, standard deviation, skewness, or kurtosis value of the elements in the cluster.

4. The method of any preceding claim, wherein the genetic identifier is the chromosome to which a gene expression represented by that element belongs, and the genetic identifier value is the chromosome number of that chromosome.

5. The method of any preceding claim, wherein a genetic identifier is the biological pathway to which a gene expression represented by that element belongs, and the genetic identifier value is a number identifying that biological pathway.

6. The method of any preceding claim, wherein a genetic identifier is the chromosome region to which the gene expression of that element belongs, and the genetic identifier value is a number identifying that region.

7. The method of any preceding claim, wherein
a first genetic identifier value and a second genetic identifier value are assigned to each element of the initial vector, and
dimensionality reduction on the initial vector is performed by referencing the first and second genetic identifier values associated with each element of the initial vector,
wherein the first and second genetic identifier values are associated with different types of genetic identifier of the gene expression of that element.

8. A method of classifying the cancer of a patient, comprising:
a. processing a cancer cell sample from the patient to obtain patient genomic data;
b. generating a lower-dimensionality patient vector, the lower-dimensionality patient vector comprising the lower-dimensionality vector generated from the patient genomic data according to the dimensionality reduction method of any previous claim; and
c. processing the lower-dimensionality patient vector using a classification model to classify the patient's cancer.

9. The method of claim 8, wherein the classification model is trained by:
a. receiving genomic data for use as training data;
b. generating lower-dimensionality training data, the lower-dimensionality training data comprising a plurality of lower-dimensionality vectors generated from the genomic training data according to the dimensionality reduction method; and
c. training the classification model using the lower-dimensionality training data.

10. A method for generating a two-dimensional representation for training a classification model , the method comprising the steps of:
receiving genomic data;
pre-processing the genomic data to generate an initial representation, each element of the initial representation comprising a gene expression;
for each element in the initial representation, assigning at least one genetic identifier value from a known set of values to the element based on at least one genetic identifier associated with a gene expression of that element;
generating a mapping matrix by referencing the at least one genetic identifier value associated with each element of the initial representation,
operating the mapping matrix on the initial representation to generate a re-arranged representation; and
outputting the re-arranged representation.

11. The method of claim 10, further comprising the step of clustering the elements of the initial representation into clusters according to the genetic identifier value assigned to each element, and
wherein the mapping matrix operates on the initial representation by re-arranging the elements of each of the clusters of the initial representation as one of:
a. a row of the re-arranged representation;
b. a column of the re-arranged representation;
c. a contiguous sub-representation within the re-arranged representation; and
d. a rectangular contiguous sub-representation within the re-arranged representation.

12. The method of claim 10 or 11, further comprising the step of clustering the elements of the initial representation into clusters according to the genetic identifier value assigned to each element, and wherein the mapping matrix operates on the initial representation by re-arranging the elements of each of the clusters of the initial representation so as to maximise the mixing of elements of different clusters within the re-arranged representation.

13. A method of classifying the cancer of a patient, comprising:
a. processing a cancer cell sample from the patient to obtain patient genomic data;
b. generating a re-arranged representation using the patient genomic data according to the re-arrangement method of any of claims 10 to 12; and
c. processing the re-arranged patient representation using a classification model to classify the patient's cancer.

14. The method of claim 13, wherein the classification model is trained by:
a. receiving genomic data for use as training data;
b. generating a re-arranged training data representation, the re-arranged training data representation comprising a plurality of re-arranged training data representations generated from the genomic training data according to the re-arrangement method; and
c. training the classification model using the re-arranged training data representation.

15. The method of any preceding claim, wherein the classification model is for classifying cancer types, optionally small cell lung cancer (SCLC) and non-small cell lung cancer (NSCLC), such as large cell lung cancer (LCLC), lung adenocarcinoma (AD) and squamous cell carcinoma (SCC) of the lung.
